# EUROPEAN PATENT APPLICATION

(11) **EP 4 413 971 A1**
(43) Date of publication of application: **14.08.2024**
(21) Application number: 24157150.4
(22) Date of filing: 12.02.2024
(51) Int. Cl.: A61K 8/31, A61K 8/34, A61K 8/42, A61K 8/9789, A61K 8/99, A61K 31/015, A61K 31/164, A61K 35/744, A61K 36/28, A61K 36/328, A61K 36/746, A61P 15/00, A61P 15/02

(54) **COMPOSITION FOR THE TREATMENT OF VULVODYNIA**

(30) Priority: 13.02.2023 IT 202300002403
(71) Applicant: TL Pharma Consulting Srl, 65129 Pescara (PE) (IT)
(72) Inventor: Torello, Giulio, Pescara (PE) (IT)
(74) Representative: IP Sextant s.r.l.

(57) **Abstract**

Cosmetic, including:
- water, in percentual weight comprised between 63.72% and 95.64%;
- Palmitoylethanolamide, in percentual weight comprised between 0.04% and 0.6%;
- freeze-dried Noni juice, in percentual weight comprised between 0.12% and 0.276%;
- β- caryophyllene, in percentual weight comprised between 0.112% and 0.24%;
- Myrrh extract, in percentual weight comprised between 0.08% and 0.12%;
- 4-t-butylcyclohexanol, in percentual weight comprised between 0.04% and 0.3%;
- glyceric extracts based on Echinacea and/or Calendula and/or Mallow and/or Chamomile, in percentage weight between 0.62% and 1.8%; and
- tyndallized lactic ferments, in percentage weight between 0.08% and 0.408%.

## Description

The present invention concerns a cosmetic for the treatment of vulvodynia.

Vulvodynia is a gynecological disease that manifests itself as a chronic condition of vulvar pain with debilitating consequences for the health and quality of life of the affected woman ^{[ 1]}. It affects up to 16% of women, without differences in age or ethnicity ^{[ 2]} and manifests itself with constant burning, itching and irritation that can spread to the entire vulva or just part of it.

The pathophysiology of Vulvodynia is currently uncertain and probably multifactorial. In fact, although inflammatory, genetic, hormonal and muscular factors are involved in the genesis of vulvodynia, the etiopathogenetic mechanisms also appear to be related to an incorrect regulation of the mechanisms responsible for the perception of pain, with an increase in nerve endings resulting from the increase in the mast cell population..

Women who suffer from it report more anxiety, fear of pain, hypervigilance, catastrophizing and depression and this can have notable consequences in their lives, with limitations in carrying out common activities of daily living ^{[ 29,30]}.

Vulvodynia also has deleterious consequences on a couple's sexuality and intimacy and overall the psychosocial component is considerably influenced.

Current products for the treatment of vulvodynia, for example described in WO 2020/016210 A1 and US 2007/0049627 A1, suffer from numerous drawbacks. The main drawback is the absence of an effective therapy that acts with a synergistic mechanism on the various factors that trigger the pathology. Not only that, the products available for treatment are not easy to find, have high costs, and are difficult to conserve as they are often galenic products, they act on the symptoms at a skin level and not at a systemic level, they can induce non-negligible unwanted side effects and they do not favor compliance, as they require repeated administration throughout the day.

There is therefore a need to improve the state of the art in reference to the therapeutic approach of this pathology and the main aim of the present invention, therefore, is to provide a product that allows vulvodynia to be treated synergistically and effectively, preventing the causes, reducing the symptoms and limiting the side effects caused by the treatment. Another purpose of the present invention is to make available an easy-to-find product for the treatment of vulvodynia, which allows women undergoing treatment to easily comply with the prescription.

The specific object of the present invention is a cosmetic, comprising:
- water, in percentual weight comprised between 63.72% and 95.64%;
- Palmitoylethanolamide, in percentual weight comprised between 0.04% and 0.6%;
- freeze-dried Noni juice, in percentual weight comprised between 0.12% and 0.276%;
- β- caryophyllene, in percentual weight comprised between 0.112% and 0.24%;
- Myrrh extract, in percentual weight comprised between 0.08% and 0.12%;
- 4-t-butylcyclohexanol, in percentual weight comprised between 0.04% and 0.3%;
- glyceric extracts based on Echinacea and/or Calendula and/or Mallow and/or Chamomile, in percentage weight between 0.62% and 1.8%; and
- tyndallized lactic ferments, in percentage weight between 0.08% and 0.408%.

According to another aspect of the invention, the cosmetic can be in the form of a cream, in which:
- said water is comprised in percentual weight comprised between 63.72% and 95.58%, optionally equal to 79.65%;
- said Palmitoylethanolamide is comprised in percentual weight comprised between 0.4% and 0.6%, optionally equal to 0.5%;
- said freeze-dried Noni juice, is included in percentage weight between 0.12% and 0.18%, optionally equal to 0.15%;
- said β-caryophyllene is included in percentage weight between 0.16% and 0.24%, optionally equal to 0.2%;
- said Myrrh extract is included in percentage weight between 0.08% and 0.12%, optionally equal to 0.1%;
- said 4-t-butylcyclohexanol is comprised in percentual weight comprised between 0.2% and 0.3%, optionally equal to 0.25%;
- said glyceric extracts based on Echinacea and/or Calendula and/or Mallow and/or Chamomile, are included in percentage weight between 1.2% and 1.08%, optionally equal to 1.5%; and
- said tyndallized lactic ferments, are included in percentage weight between 0.08% and 0.12%, optionally equal to 0.1%.

According to a further aspect of the invention, said glycerine extracts can include:
- Echinacea extract, in percentage weight between 0.4% and 0.6%, optionally equal to 0.5%;
- Calendula extract, in percentage weight between 0.4% and 0.6%, optionally equal to 0.5%;
- Mallow extract, in percentage weight between 0.2% and 0.3%, optionally equal to 0.25%; And
- Chamomile extract, in percentage weight between 0.2% and 0.3%, optionally equal to 0.25%.

According to an additional aspect of the invention, said tyndallized lactic ferments can include:
- Lactobacillus ferment lysate, in percentual weight comprised between 0.04% and 0.06%, optionally equal to 0.05%; and
- Bifida ferment lysate, in percentage weight between 0.04% and 0.06%, optionally equal to 0.05%.

According to another aspect of the invention, said cream can further comprise:
- a Phenoxyethanol and Ethylhexylglycerin based preservative, in percentage weight between 0.64% and 0.96%, optionally equal to 0.8%;
- a viscosifying compound, optionally an acrylate/C10-30 alkyl acrylate crosspolymer, in percentual weight comprised between 0.2% and 0.3%, optionally equal to 0.25%;
- Cetyl alcohol, in percentage weight between 0.4% and 0.6%, optionally equal to 0.5%;
- shea butter, in percentage weight between 1.6% and 2.4%, optionally equal to 2%;
- an emollient derived from coconut oil, in percentual weight comprised between 6.8% and 10.2%, optionally equal to 8.5%;
- a cross-linked homopolymer or acrylic acid, in percentual weight comprised between 0.08% and 0.12%, optionally equal to 0.1%;
- one octyl palmitate, in percentual weight comprised between 1.6% and 2.4%, optionally equal to 2%;
- Vegetable glycerol, in percentage weight between 2.4% and 3.6%, optionally equal to 3%;
- Lavender essential oil, in percentage weight between 0.04% and 0.06%, optionally equal to 0.05%; and
- Triethanolamine, in percentual weight comprised between 0.28% and 0.42%, optionally equal to 0.35% ;
in which the total percentage sum of all the components of the cream equals 100%.

According to a further aspect of the invention, the cosmetic can be in the form of an oil cleaner, in which:
- said water can be comprised in percentual weight comprised between 63.72% and 95.64%, optionally equal to 79.7%;
- said Palmitoylethanolamide can be included in percentage weight between 0.04% and 0.06%, optionally equal to 0.05%;

- said freeze-dried Noni juice, can be included in percentage weight between 0.184% and 0.276%, optionally equal to 0.23%;
- said β- caryophyllene can be included in percentage weight between 0.112% and 0.168%, optionally equal to 0.14%;
- said Myrrh extract can be included in percentage weight between 0.08% and 0.12%, optionally equal to 0.1%;
- said 4-t-butylcyclohexanol can be comprised in percentual weight comprised between 0.04% and 0.06%, optionally equal to 0.05%;
- said glyceric extracts based on Echinacea and/or Calendula and/or Mallow and/or Chamomile, can be included in percentage weight between 0.616% and 0.924%, optionally equal to 0.77%; and
- said tyndallized lactic ferments, can be included in percentage weight between 0.272% and 0.408%, optionally equal to 0.34%.

According to an additional aspect of the invention, said extracts can include:
- Echinacea extract, in percentage weight between 0.184% and 0.276%, optionally equal to 0.23%;
- Calendula extract, in percentage weight between 0.144% and 0.216%, optionally equal to 0.18%;
- Mallow extract, in percentage weight between 0.144% and 0.216%, optionally equal to 0.18%; and
- Chamomile extract, in percentage weight between 0.144% and 0.216%, optionally equal to 0.18%.

According to another aspect of the invention, said tyndallized lactic ferments can include:
- Lactobacillus ferment lysate, in percentual weight comprised between 0.16% and 0.24%, optionally equal to 0.2%; and
- Bifida ferment lysate, in percentage weight between 0.112% and 0.168%, optionally equal to 0.14%.

According to a further aspect of the invention, said oil cleaner can further comprise:
- a Phenoxyethanol and Ethylhexylglycerin based preservative, in percentual weight comprised between 1.464% and 2.196%, optionally equal to 1.83%;
- a viscosifying compound, optionally an acrylate/C10-30 alkyl acrylate crosspolymer, in percentual weight comprised between 0.368% and 0.552%, optionally equal to 0.46%;

- cocamidopropyl betaine, in percentage weight between 4.392% and 6.588%, optionally equal to 5.49%;
- DI-alpha tocopherol, in percentual weight comprised between 0.8% and 1.2%, optionally equal to 1%;
- Hydroxypropylcellulose, in percentual weight comprised between 0.368% and 0.552%, optionally equal to 0.46%;
- an emollient derived from olive oil, in percentual weight comprised between 2.192% and 3.288%, optionally equal to 2.74%;
- an emulsifying agent, optionally peg-120-methylglucose dioleate, in percentual weight comprised between 3.656% and 5.484%, optionally equal to 4.57%;
- a surfactant, optionally Sodium lauryl ether-7 citrate, in percentual weight comprised between 1.464% and 2.196%, optionally equal to 1.83%;
- an emulsifier, optionally Sodium Peg 7 Olive Oil Carboxylate, in percentual weight comprised between 0.24% and 0.36%, optionally equal to 0.3%; and
- Triethanolamine, in percentual weight comprised between 0.04% and 0.06%, optionally equal to 0.05%;
where the total percentage sum of all components of the oil cleaner is equal to 100%.
is also a specific object of the present invention, for use in the prevention and/or treatment of vulvodynia.

The advantages offered by the cosmetic of the present invention are significant.

In fact, the components of the cosmetic according to the invention act synergistically according to different mechanisms of action, namely:
- anti-inflammatory, analgesic, modulatory of neuropathic pain;
- modulatory of the mast cell response ;
- mood modulator, antidepressant; as well as
- local anaesthetic,
therefore, with a single product, it is possible to treat the various factors that trigger the pathology at a systemic level and, at the same time, alleviate the symptoms.

The present invention will now be described, by way of illustration and without limitation, according to its preferred embodiments.

In the following description and in the claims, the words "equal to approximately" mean "equal to" according to the usual industrial tolerances.

The cosmetic according to the invention includes:
- water, in percentual weight comprised between 63.72% and 95.64%;
- Palmitoylethanolamide, in percentual weight comprised between 0.04% and 0.6%;
- freeze-dried Noni juice, in percentual weight comprised between 0.12% and 0.276%;
- β- caryophyllene, in percentual weight comprised between 0.112% and 0.24%;
- Myrrh extract, in percentual weight comprised between 0.08% and 0.12%;
- 4-t-butylcyclohexanol, in percentual weight comprised between 0.04% and 0.3%;
- glyceric extracts based on Echinacea and/or Calendula and/or Mallow and/or Chamomile, in percentage weight between 0.62% and 1.8%; and
- tyndallized lactic ferments, in percentage weight between 0.08% and 0.408%.

Please note that of the aforementioned components, Palmitoylethanolamide is an endogenous lipid compound (amide of a fatty acid) with demonstrated analgesic and anti-inflammatory properties produced as a lipid mediator in the presence of inflammatory processes and frequently used in the treatment of neuropathic pain. In the cosmetic of the present invention it is added at a dosage that guarantees its soothing effect. It manifests its anti-inflammatory and pain-relieving action in a similar way to Cannabis, but without presenting the side effects. It works by inhibiting the release of pro-inflammatory mediators by mast cells.

Morinda citrifolia L. ( Rubiaceae ), commonly known as Noni, is a small tropical tree that grows throughout Polynesia, whose leaves, fruits, bark and roots have various pharmacological properties: antibacterial, anti-tumor and anti-inflammatory ^{[ 5]}.

β- Caryophyllene (BCP) is a sesquiterpene that acts as a natural endocannabinoid. It is present in the essential oils of spices and medicinal plants such as black pepper, rosemary, cloves etc. According to a particularly preferred embodiment of the invention, the β-caryophyllene used in the cosmetic, which guarantees the local anti-inflammatory and analgesic effect, is the one marketed under the ENDOPHYLLENE^{®} S-FL brand, by the A.C.E.F. S.p.a. company. This compound acts as a selective agonist of the CB2 cannabinoid receptors, which also exert regulatory control in the mechanisms of inflammation and in the modulation of depression and chronic pain.

As regards the Myrrh extract, this is the exudate produced by the bark of plants belonging to the Commiphora genus myrrha, a plant used for centuries as a healing and analgesic. The myrrh furanodienes curzerene, furanoeudesma-1,3-diene, and lindestrene are responsible for the analgesic activity of myrrh ^{[6]}. Myrrh can, in fact, be a therapeutic adjuvant in the presence of chronic painful neuro-inflammatory processes localized at the pelvic level and other painful syndromes. According to a particularly preferred embodiment of the invention, the Myrrh extract used in the cosmetic is known by the trade name MyrLiq ^{®}, for example marketed by the company A.C.E.F. S.p.A.. It is an extract of Commiphora myrrha with a standardized content of curzerene, furanoeudesma-1,3-diene and lindestrene (12.31 ± 0.05 g kg ⁻¹ , 18.84 ± 0.02 g kg ⁻¹ and 6.23 ± 0.01 g kg ⁻¹ , respectively), therefore a high content of total furanodienes (40.86 ± 0.78 g kg ⁻¹ ). Some studies conducted with this extract have demonstrated analgesic activity against some of the most common painful symptoms, in particular headaches, muscle pain, joint pain, lumbago, fever-related pain and menstrual cramps. A direct comparison with some of the most popular drugs frequently used (e.g. diclofenac, ketoprofen, ibuprofen, paracetamol, tramadol and ketorolac ) reveals that MyrLiq ^{®} has similar effects, although it requires a longer treatment course (20 days). In particular, painful states were significantly alleviated after treatment with 200 mg of MyrLiq ^{®}/day. No side effects were reported by the volunteers. ^{[9]}

4-t-butylcyclohexanol quickly reduces redness and skin itching and counteracts local dryness. It is a compound which, analyzed in vitro, has shown the ability to inhibit the release of prostaglandins (PGE2) and the activation of anti-inflammatory molecules (β-TNF). It also acts as an antagonist of the TRPV1 receptor (known as the capsaicin receptor and vanilloid 1 receptor) involved in the transmission and modulation of pain through the uptake of nociceptive stimuli ^{[ 7]}.

As regards the glyceric extracts based on Echinacea and/or Calendula and/or Mallow and/or Chamomile, they have a natural soothing and anti-inflammatory action.

Tyndallized lactic ferments contribute to the balance and well-being of the vaginal microbiota. In fact, in several studies, greater bacterial complexity was found in the vestibular samples of patients suffering from vulvodynia and provoked vestibulodynia (PVD). Furthermore, the researchers found that Lactobacillus gasseri was prevalent only in women with PVD, which appears to show a significant correlation with the intensity of pain/burning and severity of the disease. Associations between vulvodynia and Streptococcus, Lactobacillus gasseri or Candida have been found in other cases, as well as an overall lower number of lactobacilli in the vaginal microbiota. In line with the hypothesis of a multifactorial etiology of this syndrome, specific microbiomes may contribute to a specific vulnerability of patients ^{[ 8]}.

According to a first particularly preferred embodiment of the invention, the cosmetic is made in the form of a soothing cream, in which:
- the water is included in percentage weight between 63.72% and 95.58%, optionally equal to 79.65%;
- Palmitoylethanolamide is included in percentage weight between 0.4% and 0.6%, optionally equal to 0.5%;
- the freeze-dried Noni juice is included in percentage weight between 0.12% and 0.18%, optionally equal to 0.15%;
- β- caryophyllene is included in percentage weight between 0.16% and 0.24%, optionally equal to 0.2%;
- the Myrrh extract is included in percentage weight between 0.08% and 0.12%, optionally equal to 0.1%;
- 4-t-butylcyclohexanol is included in percentage weight between 0.2% and 0.3%, optionally equal to 0.25%;
- the glyceric extracts based on Echinacea and/or Calendula and/or Mallow and/or Chamomile are included in percentage weight between 1.2% and 1.08%, optionally equal to 1.5%; and
- the tyndallized lactic ferments are included in percentage weight between 0.08% and 0.12%, optionally equal to 0.1%.

In particular, according to a preferred embodiment of the invention, the glyceric extracts contained in the soothing cream include:
- Echinacea extract, in percentage weight between 0.4% and 0.6%, optionally equal to 0.5%;
- Calendula extract, in percentage weight between 0.4% and 0.6%, optionally equal to 0.5%;
- Mallow extract, in percentage weight between 0.2% and 0.3%, optionally equal to 0.25%; and
- Chamomile extract, in percentage weight between 0.2% and 0.3%, optionally equal to 0.25%.

As regards the tyndallized lactic ferments contained in the soothing cream of the invention, they include:
- Lactobacillus ferment lysate, in percentual weight comprised between 0.04% and 0.06%, optionally equal to 0.05%; and
- Bifida ferment lysate, in percentage weight between 0.04% and 0.06%, optionally equal to 0.05%.

In addition to the above, the soothing cream according to the first embodiment of the invention also includes:
- a Phenoxyethanol and Ethylhexylglycerin based preservative, optionally Acnibio Pe 9010 marketed by the company A.C.E.F. S.p.A., in percentage weight between 0.64% and 0.96%, optionally equal to 0.8%;
- a viscosifying compound, optionally an acrylate/C10-30 alkyl acrylate crosspolymer, in percentual weight comprised between 0.2% and 0.3%, optionally equal to 0.25%;
- cetyl alcohol, in percentage weight between 0.4% and 0.6%, optionally equal to 0.5%;
- shea butter, in percentage weight between 1.6% and 2.4%, optionally equal to 2%;
- an emollient derived from coconut oil, optionally a caprylic/capric triglyceride , in percentage weight between 6.8% and 10.2%, optionally equal to 8.5%;
- a cross-linked homopolymer or acrylic acid, optionally Carbomer 980, for example marketed by the company GENESIS SaS, in percentage weight between 0.08% and 0.12%, optionally equal to 0.1%;
- an octyl palmitate, optionally an Ethylhexyl Palmitate, in percentage weight between 1.6% and 2.4%, optionally equal to 2%; as well as
- vegetable glycerol, in percentage weight between 2.4% and 3.6%, optionally equal to 3%;
- Lavender essential oil, in percentage weight between 0.04% and 0.06%, optionally equal to 0.05%; and
- Triethanolamine, in percentual weight comprised between 0.28% and 0.42%, optionally equal to 0.35%;
in which the total percentage sum of all the components of the cream is equal to 100%.

Please note that the use of Lavender essential oil guarantees a muscle-relaxing effect and counteracts Candida strains commonly involved in infections of the oral and vaginal mucosa. Recurrent yeast infections are often the trigger for vulvodynia. Therefore, the cream of the invention is formulated for both therapeutic and preventive purposes, to limit the flare-up of the pathology.

According to a second particularly preferred embodiment of the invention, the cosmetic is made in the form of an oil cleaner, in which:
- the water is included in percentage weight between 63.72% and 95.64%, optionally equal to 79.7%;
- Palmitoylethanolamide is included in percentage weight between 0.04% and 0.06%, optionally equal to 0.05%;
- the freeze-dried Noni juice is included in percentual weight comprised between 0.184% and 0.276%, optionally equal to 0.23%;
- β- caryophyllene is included in percentage weight between 0.112% and 0.168%, optionally equal to 0.14%;
- the Myrrh extract is included in percentage weight between 0.08% and 0.12%, optionally equal to 0.1%;
- the 4-t-butylcyclohexanol is comprised in percentual weight comprised between 0.04% and 0.06%, optionally equal to 0.05%;
- the glycerine extracts based on Echinacea and/or Calendula and/or Mallow and/or Chamomile are included in percentage weight between 0.616% and 0.924%, optionally equal to 0.77%; and
- the tyndallized lactic ferments are included in percentage weight between 0.272% and 0.408%, optionally equal to 0.34%.

In the oil cleaner, according to a preferred embodiment of the invention, the glycerol extracts included are:
- Echinacea extract, in percentage weight between 0.184% and 0.276%, optionally equal to 0.23%;
- Calendula extract, in percentage weight between 0.144% and 0.216%, optionally equal to 0.18%;
- Mallow extract, in percentage weight between 0.144% and 0.216%, optionally equal to 0.18%; and
- Chamomile extract, in percentage weight between 0.144% and 0.216%, optionally equal to 0.18%.

Still in the oil cleaner of the invention, the tyndallized lactic ferments include:
- Lactobacillus ferment lysate, in percentual weight comprised between 0.16% and 0.24%, optionally equal to 0.2%; and
- Bifida ferment lysate, in percentage weight between 0.112% and 0.168%, optionally equal to 0.14%.

In addition to this, the oil cleaner according to the second embodiment of the invention includes:
- a Phenoxyethanol and Ethylhexylglycerin based preservative, optionally Acnibio Pe 9010 marketed by the company A.C.E.F. S.p.a., in percentage weight between 1.464% and 2.196%, optionally equal to 1.83%;
- a viscosifying compound, optionally an acrylate/C10-30 alkyl acrylate crosspolymer, in percentual weight comprised between 0.368% and 0.552%, optionally equal to 0.46%;
- cocamidopropyl betaine, in percentage weight between 4.392% and 6.588%, optionally equal to 5.49% ;
- DI-alpha tocopherol, in percentual weight comprised between 0.8% and 1.2%, optionally equal to 1%;
- Hydroxypropylcellulose, in percentual weight comprised between 0.368% and 0.552%, optionally equal to 0.46%;
- an emollient derived from olive oil, optionally olive oil glyceryl ether-8 esters, in percentual weight comprised between 2.192% and 3.288%, optionally equal to 2.74%;
- an emulsifying agent, optionally peg-120-methylglucose dioleate, in percentual weight comprised between 3.656% and 5.484%, optionally equal to 4.57%;
- a surfactant, optionally Sodium lauryl ether-7 citrate, in percentual weight comprised between 1.464% and 2.196%, optionally equal to 1.83%;
- an emulsifier, optionally Sodium Peg 7 Olive Oil Carboxylate, in percentual weight comprised between 0.24% and 0.36%, optionally equal to 0.3%; and
- Triethanolamine, in percentual weight comprised between 0.04% and 0.06%, optionally equal to 0.05%;
where the total percentage sum of all components of the oil cleaner is equal to 100%.

As regards the method of preparation of the aforementioned cosmetic, it should be noted that the soothing cream can advantageously be produced according to a process which includes the following operational phases:
1. add acrylate/C10-30 alkyl acrylate crosspolymer, glycerol and Carbomer 980 to the water, in the quantities indicated above, and leave to hydrate for a few minutes;
2. heat until it reaches 75°C;
3. heat an oil phase to 75°C comprising, in the quantities indicated above, cetyl alcohol, caprylic/capric triglyceride, octyl palmitate and shea butter, and add it to the mixture obtained at the end of phase 2, for example using a turboemulsifier, for example at a speed of 1800 rpm for a mixing time of approximately 2 min;
4. then cool until reaching 40°C;
5. add, in the quantities indicated above and in the order indicated, Acnibio Pe 9010, Palmitoylethanolamide, the glyceric extracts based on Echinacea and Calendula, 4-t-butylcyclohexanol, the glyceric extracts based on Mallow and Chamomile, β- caryophyllene, freeze-dried Noni juice, Myrrh extract, Lavender essential oil, Lactobacillus ferment lysate and Bifida ferment lysate, mixing, for example using a turboemulsifier, for example at a speed of 1800 rpm, for approximately 20 min.
6. neutralize the acidity of the compound thus obtained, adding triethanolamine, in the quantities indicated above, and mixing everything for approximately 20 minutes.

The oil cleaner, on the other hand, can advantageously be produced according to a process carried out at room temperature, which includes the following operating phases:
1. add the acrylate/ C10-30 alkyl acrylate crosspolymer to the water, in the quantities indicated above;
2. leave to hydrate for about 20 minutes;
3. add, in the quantities indicated above and in the order indicated, Sodium Peg 7 Olive Oil Carboxylate , cocamidopropyl betaine, peg-120-methylglucose dioleate, olive oil glyceryl ether-8 esters, Sodium lauryl ether-7 citrate, Acnibium Pe 9010, Alpha tocopherol, hydroxypropylcellulose, Palmitoylethanolamide, 4-t-butylcyclohexanol, freeze-dried Noni juice, glyceric extracts based on Echinacea , Calendula, Mallow and Chamomile, β- caryophyllene , the Lactobacillus ferment lysate and the Bifida ferment lysate and the Myrrh extract, mixing for about 20 minutes;
4. neutralize the acidity of the compound thus obtained, adding triethanolamine, in the quantities indicated above, and mixing everything for about 20 minutes.

The cosmetic of the invention is advantageously used for use in the prevention and/or treatment of vulvodynia and can be used according to a dosage that facilitates compliance. In particular, the oily cleanser is to be used for personal hygiene and the soothing cream can be applied 1 or 2 times a day.

Preliminary tests demonstrated the beneficial effects of the detergent and cream according to the invention.

In particular, an in vitro comparative test was conducted to demonstrate the potential of the claimed cosmetic, with a specific effect on pathogenic bacteria and fungi, recognized among the factors triggering vulvodynia, and on the protection of the vaginal bacterial flora, the alteration of which is also it is related to an increased risk of vulvovaginal diseases.

The test was conducted in the following ways.

The claimed cosmetic was applied to a culture of Candida albicans ATCC^{®} (American Type Culture Collection) 10231 for the evaluation of the efficacy against pathogenic fungi, to a culture of Escherichia coli ATCC^{®} 25922 for the evaluation of the efficacy against of bacteria. Furthermore, the claimed cosmetic was applied to a culture of Lactobacillus rhamnosus obtained from ATCC^{®} 7469^{™} to evaluate its effects on the protection of the bacterial flora.

All of the above strains of microorganisms were subjected to antimicrobial susceptibility testing using the Kirby-Bauer disk diffusion method as described by National Committee on Clinical Laboratory Standards Guidelines, 1993 (NCCLS). The minimum inhibitory concentration (MIC) is determined by the microdilution method ^{[ 10]}. The data are evaluated using IBM SPSS software program (version 19; IBM SPSS Inc., IL USA). All samples and control groups are compared with a 95% confidence interval. Culture and antimicrobial assay details are described in ^{[11]}.

The claimed cosmetic applied to such strains of microorganisms is in particular the following:
- water in percentual weight comprised at 94.056%;
- palmitoylethanolamide in percentual weight comprised at 0.6%;
- freeze-dried Noni juice in percentual weight comprised at 0.276%;
- β- caryophyllene in percentual weight comprised at 0.24%;
- and Myrrh extract in percentual weight comprised at 0.12%;
- 4-t-butylcyclohexanol in percentual weight comprised at 0.3%;
- glycerine extracts based on Echinacea in percentual weight comprised at 1.8%; and
- tyndallized lactic ferments in percentual weight comprised at 0.408%.

For the purpose of comparison, a comparative product, as described in international patent application WO 2020/016210, was prepared and then applied to a culture of Candida albicans ATCC^{®} 10231, to a culture of Escherichia coli ATCC^{®} 25922, and to a Lactobacillus rhamnosus culture obtained from ATCC^{®} 7469^{™}.

In detail, the comparative product provides, as indicated in document WO 2020/016210, the following composition:
- water in percentual weight comprised at 97.05%;
- calendula officinalis flower oil in percentual weight comprised at 0.25%;
- curcuma longa root extract in percentual weight comprised at 0.25%; And
- Eugenia caryophyllus bud extract in percentual weight comprised at 0.25%.

For completeness of the formulation, preservatives, neutralizers and pH regulators were added in a weight percentage equal to 2.2%.

The test results demonstrated that the cosmetic has greater selectivity of action, compared to the product obtained from the teaching of document WO 2020/ 016210, against pathogenic microorganisms (in particular Candida albicans ATCC^{®} 10231 and Escherichia coli ATCC^{®} 25922) without modifying the population of lactobacilli (in particular Lactobacillus rhamnosus obtained from ATCC^{®} 7469^{™}) present in the application area.

The testing methodology described above is based on the following considerations.

intestinal permeability ^{[ 12]}. It is therefore necessary to control the growth of this microorganism in order to counteract vulvodynia.

Observational studies also demonstrate that a significant number of women with vulvodynia had suffered from vaginal candidiasis. These patients, although undergoing treatment for candidiasis, later developed symptoms of chronic vulvodynia ^{[ 13]}. It follows that to combat vulvodynia it is important to control the growth of this fungus.

The International Society for the Study of Vulvovaginal Disease also assumes a correlation between alteration of the vaginal microbiota and increased risk of vulvovaginal diseases, since a lower number of lactobacilli has been found in the vaginal microbiota of patients suffering from vulvodynia and, given the multifactorial etiology of the syndrome, alterations in the microbiota can contribute to a patient's predisposition to manifest vulvodynia ^{[ 14]}. Furthermore, it is known that the population of lactobacilli is a natural barrier against the attacks of pathogenic microorganisms ^{[ 15]}. For this reason it is therefore appropriate that to counteract vulvodynia a cosmetic safeguards as much as possible the lactic bacteria fundamental for vaginal homeostasis, since the reduction of the population of bacteria that maintain the slightly acidic vaginal pH, as they produce lactic acid, certainly reduces the protective effect for which these bacteria are responsible ^{[ 16]}.

In addition to the in vitro comparative test, a clinical test was performed on 17 subjects, resulting in, with a power of 80%, a probability of type I alpha error equal to 0.05 and with an effect size (d) equal to 0.827, that the minimum numerousness was equal to 15 subjects.

The subjects were selected according to the following inclusion criteria:
- female sex;
- aged between 18 and 70;
- general state of good health;
- diagnosis of vulvodynia; and
- subjective evaluation of dyspaurenia greater than or equal to 2 on the VAS scale.

The following were excluded:
- subjects who are pregnant, breastfeeding or planning to become pregnant before the end of the study;
- subjects with other concomitant pathologies such as cancer, fibromyalgia, chronic intestinal inflammatory diseases, autoimmune diseases;
- subjects with proven sensitivity to one of the components;
- subjects who require the prescription of a new therapy compared to the one already underway at the time of enrollment;
- subjects who have any other condition that, in the opinion of the investigator, could compromise the safety or availability of the subject or adherence to the study protocol or could interfere with the interpretation of the results and would therefore make the subject inappropriate for the study inclusion in monitoring; and
- subjects who are not self-sufficient in the application of the medical device.

The clinical study lasted 18 weeks starting from a first visit in which the following occurred: baseline visit, enrollment, collection of self-assessments expressed via VAS (Visual Analogue Scale), completion of the SF-36 questionnaire (quality assessment questionnaire of life) for the quality of life, and assignment of treatment with the claimed cosmetic.

Subsequently, 60 days after the start of treatment with the claimed cosmetic, the self-assessments expressed via VAS scale were collected, the SF-36 questionnaire for quality of life was completed and any adverse events were recorded.

At the end of 18 weeks from the start of treatment with the claimed cosmetic, the self-assessments expressed via VAS scale were collected, the SF-36 questionnaire for quality of life was completed and any adverse events were recorded.

The self-assessments include: self-assessment of dyspaurenia on a VAS scale from 0 to 10, self-assessment of spontaneous vestibular pain on a VAS scale from 0 to 10, self-assessment of provoked vestibular pain on a VAS scale from 0 to 10, self-assessment of symptoms from vulvo -vaginal infection in VAS scale from 0 to 10.

The clinical tests showed for the sample subjected to treatment with the claimed cosmetic a reduction in the value on the VAS scale in the self-assessment of dyspaurenia, a reduction in the value on the VAS scale in the self-assessment of spontaneous vestibular pain, a reduction in the value on the VAS in the self-assessment of provoked vestibular pain, a reduction in the VAS scale value in the self-assessment of symptoms from vulvo-vaginal infection, and an improvement in the quality of life based on the completion of the SF-36 questionnaire.

In the foregoing, the preferred embodiments have been described and variations of the present invention have been suggested, but it is to be understood that those skilled in the art will be able to make modifications and changes without thereby departing from the relevant scope of protection, as defined by the claims. attached.

### BIBLIOGRAPHY

[1]. Rosen NO, Dawson SJ, Brooks M., Kellogg-Spadt S.; Treatment of Vulvodynia: Pharmacological and Non-pharmacological Approaches, Springer Nature Switzerland, 2019, 79:483-493.
[2]. Vasileva P., Strashilov SA, Yordanov AD; Aetiology, diagnosis, and clinical management of vulvodynia, Menopause Rev 2020; 19(1): 44-48.
[3] Arnold, L.D.; Bachmann, G.A.; Rosen, R.; Kelly, S.; Rhoads, GG Vulvodynia: Characteristics and associations with comorbidities and quality of life. Obstet. Gynecol. 2006, 107, 617-624.
[4] Latthe , P.; Mignini, L.; Gray, R.; Hills, R.; Khan, K. Factors predisposing women to chronic pelvic pain: Systematic review. BMJ 2006, 332, 749-755.
[5] Dussossoy , E.; Brat, P.; Bony, E.; Boudard , F.; Poucheret , P.; Mertz, C.; Giaimis , J.; Michel, A. Characterization, Anti-Oxidative and Anti-Inflammatory Effects of Costa Rican Noni Juice ( Morinda citrifolia L.). J. Ethnopharmacol . 2011, 133, 108-115.
[6] P. Dolara , C. Luceri , C. Ghelardini et al., "Analgesic effects of myrrh [5]," Nature, vol. 379, no. 6560, p. 29, 1996.
[7] Sulzberger, M.; Worthmann, A.-C.; Holtzmann, U.; Buck, B.; Jung, K.A.; Schoelermann , A.M.; Rippke, F.; Stab, F.; Wenck, H.; Neufang, G.; Gronniger, E. Effective treatment for sensitive skin: 4-t-butylcyclohexanol and licochalcone A. Journal of the European Academy of Dermatology & Venereology. 30 ( Supplement 1):9-17, February 2016.
[8] Francesco De Seta, MD, Risa Lonnee -Hoffmann Giuseppina Campisciano , Manola Comar , Hans Verstraelen , Pedro Vieira-Baptista, Gary Ventolini, and Ahinoam Lev- Sagie . The Vaginal Microbiome: III. The Vaginal Microbiome in Various Urogenital Disorders, Journal of Lower Genital Tract Disease • Volume 26, Number 1, January 2022
[9] Germano A., Occhipinti A., Barbero F., Maffei ME A Pilot Study on Bioactive Constituents and Analgesic Effects of MyrLiq, a Commiphora myrrha Extract with a High Furanodiene Content, Clinical Study, BioMed Research International, Volume 2017, Article ID 3804356, 11 pages, https://doi.org/10.1155/2017/3804356
[10] Swenson JM, George E, Killgore FC: Tenover , Antimicrobial Susceptibility Testing of Acinetobacter spp. by NCCLS Broth Microdilution and Disk Diffusion Methods. J. Clin . Microbiol ., 2004, 42, 5102-5108.
[11] Micucci M, Gotti R, Corazza I, Tocci G, Chiarini A, De Giorgio M, Camarda L, Frosini M, Marzetti C, Cevenini M, Budriesi R. Newer Insights Into the Antidiarrheal Effects of Acacia Catechu Willd . Extract in Guinea Pig. J Med Food, 2016, 20, 592-600.
[12] Coda L, Cassis P, Angioletti S, Angeloni C, Piloni S, Testa C. Evaluation of Gut Microbiota in Patients With Vulvovestibular Syndrome. J Clin Med Res. 2021 Feb;13(2):101-106.
[13] Ramirez De Knott HM, McCormick TS, Do SO, Goodman W, Ghannoum MA, Cooper KD, Nedorost ST. Cutaneous hypersensitivity to Candida albicans in idiopathic vulvodynia. Contact Dermatitis. 2005 Oct;53(4):214-8.
[14] De Seta F, Lonnee-Hoffmann R, Campisciano G, Comar M, Verstraelen H, Vieira-Baptista P, Ventolini G, Lev-Sagie A. The Vaginal Microbiome: III. The Vaginal Microbiome in Various Urogenital Disorders. J Low Genit Tract Dis. 2022 Jan 1;26(1):85-92.
[15] Vadala M, Testa C, Coda L, Angioletti S, Giuberti R, Laurino C, Palmieri B. Vulvovestibular Syndrome and Vaginal Microbiome: A Simple Evaluation. J Clin Med Res. 2018 Sep;10(9):688-692.
[16] De Seta F, Lonnee -Hoffmann R, Campisciano G, Comar M, Verstraelen H, Vieira-Baptista P, Ventolini G, Lev- Sagie A. The Vaginal Microbiome : III. The Vaginal Microbiome in Various Urogenital Disorders. J Low Genit Tract Dis. 2022 Jan 1;26(1):85-92.

## Claims

1. Cosmetic, including:
- water, in percentual weight comprised between 63.72% and 95.64%;
- Palmitoylethanolamide, in percentual weight comprised between 0.04% and 0.6%;
- freeze-dried Noni juice, in percentual weight comprised between 0.12% and 0.276%;
- β- caryophyllene, in percentual weight comprised between 0.112% and 0.24%;
- Myrrh extract, in percentual weight comprised between 0.08% and 0.12%;
- 4-t-butylcyclohexanol, in percentual weight comprised between 0.04% and 0.3%;
- glyceric extracts based on Echinacea and/or Calendula and/or Mallow and/or Chamomile, in percentage weight between 0.62% and 1.8%; and
- tyndallized lactic ferments, in percentage weight between 0.08% and 0.408%.

2. Cosmetic according to claim 1, in the form of a cream, in which:
- said water is comprised in percentual weight comprised between 63.72% and 95.58%, optionally equal to 79.65%;
- said Palmitoylethanolamide is comprised in percentual weight comprised between 0.4% and 0.6%, optionally equal to 0.5%;
- called freeze-dried Noni juice, is included in percentage weight between 0.12% and 0.18%, optionally equal to 0.15%;
- said β-caryophyllene is included in percentage weight between 0.16% and 0.24%, optionally equal to 0.2%;
- said Myrrh extract is included in percentage weight between 0.08% and 0.12%, optionally equal to 0.1%;
- said 4-t-butylcyclohexanol is comprised in percentual weight comprised between 0.2% and 0.3%, optionally equal to 0.25%;
- said glyceric extracts based on Echinacea and/or Calendula and/or Mallow and/or Chamomile, are included in percentage weight between 1.2% and 1.08%, optionally equal to 1.5%; and
- called tyndallized lactic ferments, are included in percentage weight between 0.08% and 0.12%, optionally equal to 0.1%.

3. Cosmetic according to claim 2, wherein said glyceric extracts comprise:
- Echinacea extract, in percentage weight between 0.4% and 0.6%, optionally equal to 0.5%;
- Calendula extract, in percentage weight between 0.4% and 0.6%, optionally equal to 0.5%;
- Mallow extract, in percentage weight between 0.2% and 0.3%, optionally equal to 0.25%; and
- Chamomile extract, in percentage weight between 0.2% and 0.3%, optionally equal to 0.25%.

4. Cosmetic, according to claim 2 or 3, wherein said tyndallized lactic ferments comprise:
- Lactobacillus ferment lysate, in percentual weight comprised between 0.04% and 0.06%, optionally equal to 0.05%; and
- Bifida ferment lysate, in percentage weight between 0.04% and 0.06%, optionally equal to 0.05%.

5. Cosmetic according to any one of claims 2 to 4, wherein said cream further comprises:
- a Phenoxyethanol and Ethylhexylglycerin based preservative, in percentual weight comprised between 0,64% and 0,96%, optionally equal to 0,8%;
- a viscosifying compound, optionally an acrylate/C10-30 alkyl acrylate crosspolymer, in percentual weight comprised between 0,2% and 0,3%, optionally equal to 0,25%;
- Cetyl Alcohol, in percentual weight comprised between 0,4% and 0,6%, optionally equal to 0,5%;
- shea butter, in percentual weight comprised between 1,6% and 2,4%, optionally equal to 2%;
- an emollient derived from coconut oil, in percentual weight comprised between 6.8% and 10.2%, optionally equal to 8.5%;
- a cross-linked homopolymer or acrylic acid, in percentual weight comprised between 0.08% and 0.12%, optionally equal to 0.1%;
- an octyl palmitate, in percentual weight comprised between 1.6% and 2.4%, optionally equal to 2%;
- vegetable glycerin, in percentage weight between 2.4% and 3.6%, optionally equal to 3%;
- Lavender essential oil, in percentage weight between 0.04% and 0.06%, optionally equal to 0.05%; and
- Triethanolamine, in percentual weight comprised between 0.28% and 0.42%, optionally equal to 0.35% ;
in which the total percentage sum of all the components of the cream equals 100%.

6. Cosmetic according to claim 1, in the form of an oil cleaner, wherein:
- said water is included in percentage weight between 63.72% and 95.64%, optionally equal to 79.7%;
- said Palmitoylethanolamide is comprised in percentual weight comprised between 0.04% and 0.06%, optionally equal to 0.05%;
- said freeze-dried Noni juice, is included in percentage weight between 0.184% and 0.276%, optionally equal to 0.23%;
- said β-caryophyllene is included in percentage weight between 0.112% and 0.168%, optionally equal to 0.14%;
- said Myrrh extract is included in percentage weight between 0.08% and 0.12%, optionally equal to 0.1%;
- said 4-t-butylcyclohexanol is comprised in percentual weight comprised between 0.04% and 0.06%, optionally equal to 0.05%;
- said glyceric extracts based on Echinacea and/or Calendula and/or Mallow and/or Chamomile, are included in percentage weight between 0.616% and 0.924%, optionally equal to 0.77%; and
- said tyndallized lactic ferments are comprised in percentual weight between 0,272% and 0,408%, optionally equal to 0,34%.

7. Cosmetic according to claim 6, wherein said extracts comprise:
- Echinacea extract, in percentage weight between 0.184% and 0.276%, optionally equal to 0.23%;
- Calendula extract, in percentage weight between 0.144% and 0.216%, optionally equal to 0.18%;
- Mallow extract, in percentage weight between 0.144% and 0.216%, optionally equal to 0.18%; and
- Chamomile extract, in percentage weight between 0.144% and 0.216%, optionally equal to 0.18%.

8. Cosmetic, according to claim 6 or 7, wherein said tyndallized lactic ferments comprise:
- Lactobacillus ferment lysate, in percentual weight comprised between 0.16% and 0.24%, optionally equal to 0.2%; and
- Bifida ferment lysate, in percentage weight between 0.112% and 0.168%, optionally equal to 0.14%.

9. Cosmetic according to any one of claims 6 to 8, wherein said oil cleaner further comprises:
- a Phenoxyethanol and Ethylhexylglycerin based preservative, in percentual weight comprised between 1.464% and 2.196%, optionally equal to 1.83%;
- a viscosifying compound, optionally an acrylate/C10-30 alkyl acrylate crosspolymer, in percentual weight comprised between 0.368% and 0.552%, optionally equal to 0.46%;
- cocamidopropyl betaine, in percentage weight between 4.392% and 6.588%, optionally equal to 5.49%;
- DI-alpha-Tocopherol, in percentual weight comprised between 0.8% and 1.2%, optionally equal to 1%;
- Hydroxypropylcellulose, in percentual weight comprised between 0.368% and 0.552%, optionally equal to 0.46%;
- an emollient derived from olive oil, in percentual weight comprised between 2.192% and 3.288%, optionally equal to 2.74%;
- an emulsifying agent, optionally peg-120-methylglucose dioleate, in percentual weight comprised between 3.656% and 5.484%, optionally equal to 4.57%;
- a surfactant, optionally Sodium lauryl ether-7 citrate, in percentual weight comprised between 1.464% and 2.196%, optionally equal to 1.83%;
- an emulsifier, optionally Sodium Peg-7 Olive Oil Carboxylate, in percentual weight comprised between 0.24% and 0.36%, optionally equal to 0.3%; and
- Triethanolamine, in percentual weight comprised between 0.04% and 0.06%, optionally equal to 0.05% ;
where the total percentage sum of all components of the oil cleaner is equal to 100%.

10. Cosmetic according to any of the preceding claims, for use in the prevention and/or treatment of vulvodynia.
